# EUROPEAN PATENT APPLICATION

(11) **EP 4 717 221 A1**
(43) Date of publication of application: **01.04.2026**
(21) Application number: 25204781.6
(22) Date of filing: 25.09.2025
(51) Int. Cl.: A61B 34/20, A61B 17/84, A61B 17/00

(54) **MEDICAL INSTRUMENT**

(30) Priority: 25.09.2024 US 202463698703 P; 25.09.2024 EP 24202557
(71) Applicant: Fiagon GmbH, 16761 Hennigsdorf (DE)
(72) Inventor: Hedermann, Robert, 10409 Berlin (DE); Kersten, Lutz, 12163 Berlin (DE)
(74) Representative: Eisenführ Speiser

(57) **Abstract**

The invention relates to a Kirschner-wire (10) comprising a localizer coil (20) for an electromagnetic navigation system. The localizer coil (20) is arranged in a distal section of the K-wire (10) at a distance of between 4 mm and 24 mm from a distal tip (14) of the K-wire (10). The localizer coil (20) is electrically connected to two electrically conducting signal lines (22, 24) for passing electric signals from the localizer coil (20) to an electromagnetic navigation system. The electrically conducting signal lines (22, 24) comprise a releasable and reconnectable electric and mechanical connector (30) that allows to disconnect and reconnect distal sections (22.1, 24.1) of the electrically conducting signal lines (22, 24) from proximal sections (22.2, 24.2) of said electrically conducting signal lines (22, 24). The connector (30) is arranged near or at a proximal end of the K-wire (10) such that a cannulated instrument can be slid over the K-wire (10) when the proximal sections (22.2, 24.2) are electrically and mechanically disconnected from the respective distal sections (22.1, 24.1) of the electrically conducting signal lines (22, 24).

## Description

### Background

The invention relates to a medical instrument with a wire for penetrating body tissue.

For surgery, Kirschner wires - also called K-wires - are used for marking an access and guiding a cannulated instrument to a region of interest. K-wires typically have a sharpened tip and are made from bio-compatible metal such as stainless steel or titanium alloy. Due to their small diameter of between 0.4 mm and 3 mm, K-wires are flexible and may bend during use. The sharp tip may have a thread, i.e. is threaded or may have a trocar tip with three bevel planes and cutting edges forming a sharp point.

Due to bending of the K-wire, precise positioning of the k-wire tip is difficult.

### Summary

It is an object of the invention to provide a K-wire that facilitates handling.

In a first aspect, the invention provides a Kirschner-wire comprising a proximal end, a distal tip and a localizer coil for an for an electromagnetic navigation system.

The localizer coil is arranged in a distal section of the Kirschner-wire at a distance of between about 4 mm and about 24 mm from the distal tip. The localizer coil is electrically connected to at least a first electrically conducting signal line and a second electrically conducting signal line for passing at least a first electric signal and a second electric signal from the localizer coil to an electromagnetic navigation system. Each of the first electrically conducting signal line and the second electrically conducting signal line comprises a releasable and reconnectable electric and mechanical connector that allows disconnection and reconnection of a distal section of the first electrically conducting signal line and the second electrically conducting signal line from a proximal section of each of said first electrically conducting signal line and the second electrically conducting signal line. A connector is arranged near or at the proximal end of the Kirschner-wire for allowing a cannulated instrument being slid over the Kirschner-wire when the proximal sections of the first electrically conducting signal line and a second electrically conducting signal line are electrically and mechanically disconnected from the respective distal sections of the first electrically conducting signal line and a second electrically conducting signal line.

In various embodiments, the connector comprises at least a first electrically conducting ring contact element and a second electrically conducting ring contact element. The first electrically conducting ring contact element and the second electrically conducting ring contact element are arranged at or near to the proximal end of the Kirschner-wire. The first electrically conducting ring contact element and the second electrically conducting ring contact element are spaced apart from each other in a longitudinal direction of the Kirschner-wire and are electrically insulated from the Kirschner-wire. Both, the first electrically conducting ring contact element and the second electrically conducting ring contact element are electrically connected to a respective one of the proximal sections of the first electrically conducting signal line and the second electrically conducting signal line.

According to the invention, a k-wire is provided, wherein a localizer coil configured for a use in an electromagnetic navigation system is arranged in a distal region of the k-wire wherein the distance between a distal end of the localizer coil and the distal end of the k-wire is between 2 mm and 24 mm. The localizer coil is connected to electrically conducting signal lines for electrically connecting the localizer coil to an electromagnetic navigation system. According to the invention, a releasable electric and mechanical connection is provided that allows mechanically disconnecting electrically conducting signal lines from the sensor coil. The electrically conducting signal lines comprise a releasable and reconnectable electric and mechanical connector that allows to disconnect and reconnect distal sections of the electrically conducting signal lines from proximal sections of the electrically conducting signal lines. The connector is arranged near or at a proximal end of the K- wire such that a cannulated instrument can be slid over the K-wire when the proximal sections are electrically and mechanically disconnected from the respective distal sections of the electrically conducting signal lines.

According to one embodiment, the releasable electrical and mechanical connection for the electrically conducting signal lines is arranged at a distance of between 4 cm and 20 cm from the localizer coil. Accordingly, a distal section of the electrically conducting signal line is non-releasably connected to the localizer coil, while a proximal section of the electrically conducting signal line can be electrically and mechanically released from and reconnected to the distal section of the electrically conducting signal line.

The connector, in some embodiments, comprises two electrically conducting ring contact elements that are arranged at or close to the proximal end of the Kirschner- wire. The ring contact elements may be spaced apart from each other in a longitudinal direction of the Kirschner-wire and are electrically insulated from the Kirschner-wire. Each ring contact element is, in some embodiments, electrically connected to a respective one of the proximal sections of the electrically conducting signal lines.

In some embodiments, the Kirschner-wire is hollow and has a lumen wherein the localizer coil is arranged within said lumen.

In some embodiments, the Kirschner-wire has an outer diameter of between about 1.5 mm and about 2.5 mm.

The localizer coil may comprise an outer diameter of less than about 1 mm, or less than about 0.5 mm, or less than about 0.45 mm. In some embodiments, the localization coil comprises an outer diameter of about 0.45 mm.

In one embodiment, the localizer coil is wound around the k-wire. Further, in some embodiments, if the distal, non-releasable sections of the electrically conducting signal lines are arranged along the k-wire. Thus, the localizer coil and the distal sections of the electrically conducting signal lines do not add much to the diameter of the k-wire. Thus, when the proximal sections of the electrically conducting signal lines are disconnected from the distal sections of the electrically conducting signal lines, a cannulated instrument can be slid over the k-wire while the sensor coil is permanently attached to the k-wire.

### Brief Description of the Drawings

The invention and further aspects shall now be described by way of exemplary embodiments with reference to the figures. In the figures
- Fig. 1A:: is a schematic view of a distal end section of a k-wire with a localizer coil and electrically conducting signal lines
- Fig. 1B:: is a schematic, enlarged view of a part of a distal section of the k-wire illustrated in figure 1A;
- Fig. 2A:: is a schematic view of the k-wire of figure 1A without localizer coil and electrically conducting signal lines;
- Fig. 2B:: is a schematic view of the localizer coil and the electrically conducting signal lines without the k-wire from figure 1A; and
- Figs. 3A and B:: are schematic illustrations of a releasable electrical and mechanical connection between distal sections and proximal sections of the electrically conducting signal lines; and
- Fig. 4:: is a schematic illustration of a hollow k-wire with a lumen in which the localizer coil is arranged; and
- Fig. 5:: is a schematic illustration of an alternative embodiment of a hollow k-wire with a lumen in which the localizer coil is arranged.

### Detailed Description

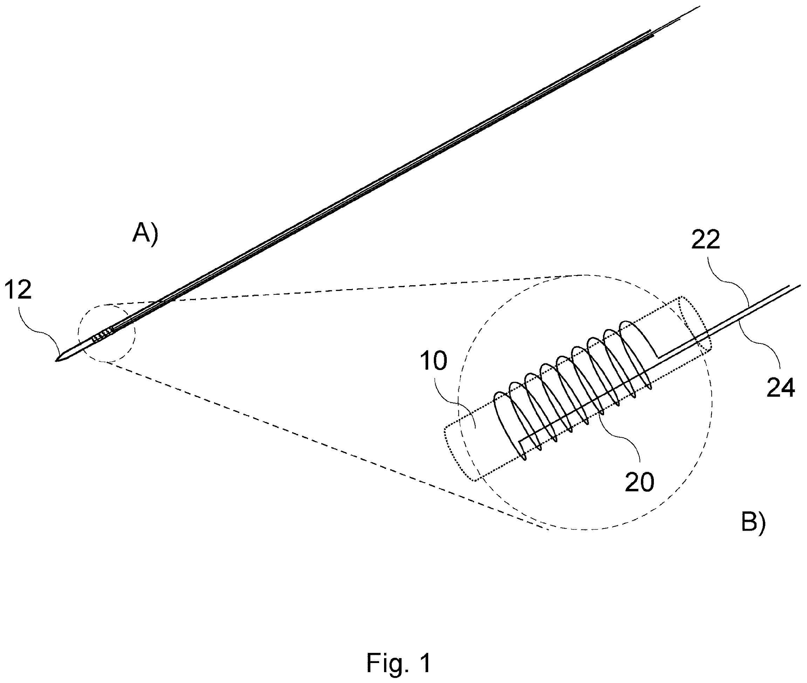

As shown in figure 1A, a distal section of a k-wire 10 is provided with a localizer coil 20 that is arranged at a distance of between 2 mm and 24 mm from a distal tip 12 of the k-wire 10. The distal tip region of the k-wire 10 is sharpened and may be trocar grinded.

The localizer coil 20 is electrically connected with two electrically conducting signal lines 22 and 24 for feeding electric signals from the localizer coil 20 to an electromagnetic navigation system.

The enlarged view in figure 1B illustrates that the localizer coil 20 is wound around the k-wire 10 and that the electrically conducting signal lines 22 and 24 are arranged along the length of the k-wire 10.

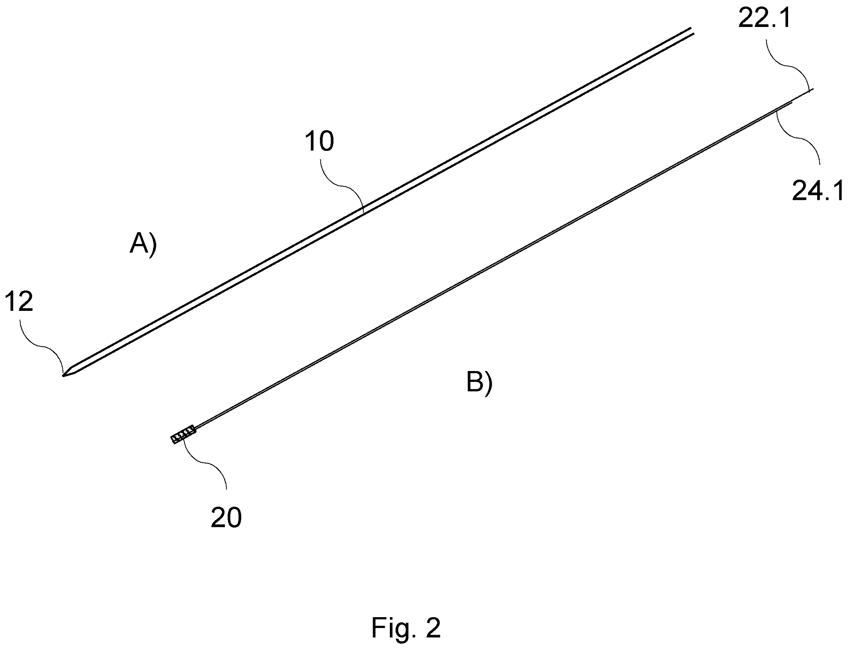

Figure 2A shows the k-wire 10 without the localizer coil 20 and its electrically conducting signal lines 22 and 24 while figure 2B shows the localizer coil 20 and its electrically conducting signal lines 22 and 24 without the k-wire 10.

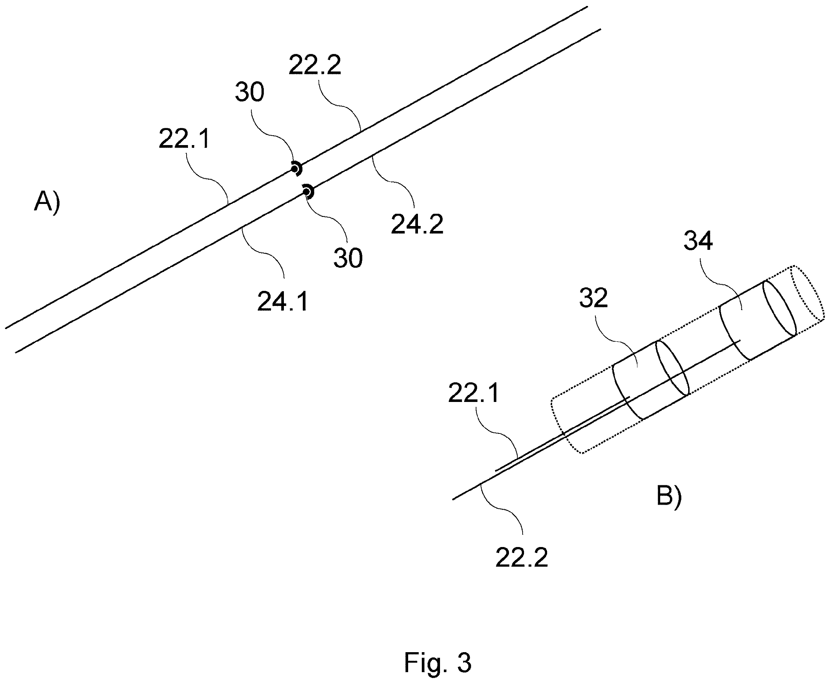

As illustrated in figure 3 the electrically conducting signal lines 22 and 24 are each having a distal section 22.1 and 24.1, respectively and a proximal section and a proximal signal line section 22.2 and 24.2 respectively. The proximal signal line sections 22.1 and 24.1 are each electrically and mechanically connected to the respective proximal signal line sections 22.2 and 24.2 by means of a connector 30.

As shown in figure 3, the connector 30 providing a releasable mechanical and electric connection between the distal sections 22.1 and 24.1 of the electrically conducting signal lines and the respective proximal sections 22.2 and 22.4 of the electrically conducting signal lines may comprise electrically conducting contact elements 32 and 34 that are arranged near a proximal end of the K-wire 10. The contact elements 32 and 34 may be electrically conducting rings arranged about the outer circumferential surface of the K-wire 10. Each electrically conducting contact element 32 and 34 is electrically connected to one of the distal sections 22.1 and 24.1 of the electrically conducting signal lines 22 and 24. The electrically conducting ring contact elements 32 and 34 are spaced from each other in an axial direction of the K-wire 10. Further, the electrically conducting ring contact elements 32 and 34 are electrically isolated from the K-wire 10. For instance, an insulation layer may be provided between the outer surface of the K-wire 10 and the inner surfaces of the ring contact elements 32 and 34.

The proximal sections 22.2 and 24.2 of the electrically conducting signal lines 22 and 24 may be connected to a contact sleeve (not shown) that can be slid over the proximal end of the K-wire 10 and the ring contact elements 32 and 34. Within the sleeve, electric contacts may be provided that are configured and arranged to contact the ring contact element 32 and the ring contact element 34, respectively.

Once the localizer coil 20 is connected to an electromagnetic navigation system, a precise positioning of the K-wire can be facilitated by means of electromagnetic navigation on the localizer coil 20 is electrically connected to an EM navigation system via the electrically conducting signal lines 22 and 24. This is the case, when the proximal sections 22.2. and 24.2 of the electrically conducting signal lines 22 and 24 are connected to the respective distal sections 22.1 and 24.1 of the electrically conducting signal lines via the contact sleeve and the contact ring elements 32 and 34. Due to the wired connection between the localizer coil 20 and the EM navigation system, no cannulated instrument can be slid over the K-wire while the localizer coil 20 is connected to the electromagnetic navigation system. However, once the K-wire is precisely placed and fixated, the electric and mechanical connection between the distal sections 22.1 and 24.1 and the proximal sections 22.2 and 24.2 of the electrically conducting signal lines 22 and 24 can be released to thus allow a cannulated instrument to be slid over the K-wire.

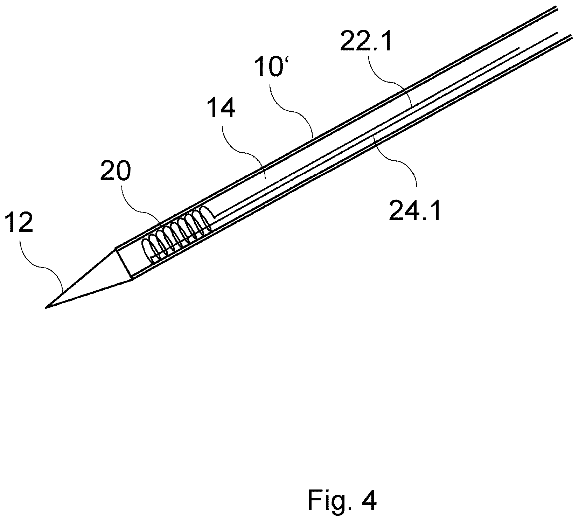

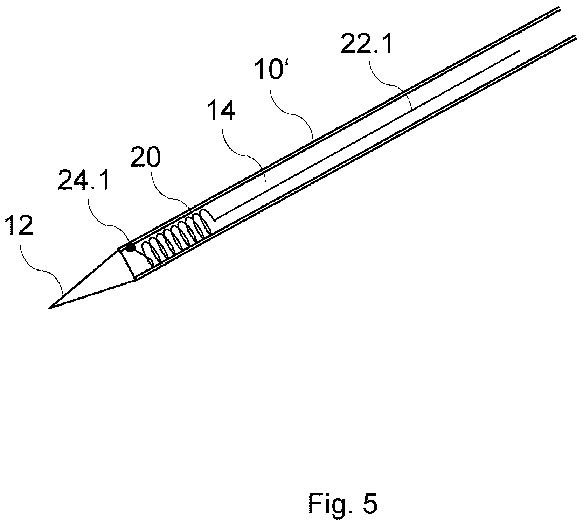

In alternative embodiments as illustrated in figures 4 and 5, the K-wire 10' may be hollow and comprises a lumen 14. With such a K-wire 10', the localizer coil 20 may be arranged within the lumen 14 of the K-wire 10' as illustrated in figures 4 and 5. In such embodiment, a tip portion 12' of the K-wire 10' may be made of solid metal and may be welded or brazed to the rest of the K-wire 10'. In other words: the K-wire 10' is made from a tube and a solid tip portion that are connected to each other to form an integral member. The distal signal line sections 22.1 and 24.1 may also run inside the lumen 14 of the K-wire 10' and may be connected to the ring contact elements 32 and 34; see figure 4.

In an alternative embodiment as shown in figure 5, only one distal signal line section 22.1 is embodied as a wire that extends between the localizer coil 20 and the respective ring contact element. The other distal signal line section 24.1 is formed by the outer metallic wall 18 of the K-wire 10'.

The localizer coil 20 is a five degrees of freedom (5 DoF) position and orientation sensor and may have an outer diameter of less than about 1 mm or even less than about 0.5 mm, for instance 0.45 mm. The K-wire 10 may comprise an outer diameter of about 2 mm, for instance an outer diameter of between about 1.5 mm and about 2.5 mm.

### Reference numerals:

- 10: k-wire
- 12: distal tip of the k-wire
- 14: lumen of the k-wire
- 18: metallic outer, circumferential wall of the k-wire
- 20: localizer coil
- 22 and 24: electrically conducting signal lines
- 22.1 and 24.1: distal (signal line) sections
- 22.2 and 24.2: proximal (signal line) sections
- 30: connector
- 32 and 34: ring contact elements

## Claims

1. Kirschner-wire (10) comprising a localizer coil (20) for an electromagnetic navigation system, wherein the localizer coil (20) is arranged in a distal section of the Kirschner-wire (10) at a distance of between 4 mm and 24 mm from a distal tip (14) of the Kirschner-wire (10), and wherein the localizer coil (20) is electrically connected to two electrically conducting signal lines (22, 24) for passing electric signals from the localizer coil (20) to an electromagnetic navigation system, said electrically conducting signal lines (22, 24) comprising a releasable and reconnectable electric and mechanical connector (30) that allows to disconnect and reconnect distal sections (22.1, 24.1) of the electrically conducting signal lines (22, 24) from proximal sections (22.2, 24.2) of said electrically conducting signal lines (22, 24) wherein the connector (30) is arranged near or at a proximal end of the Kirschner-wire (10) and wherein a cannulated instrument can be slid over the Kirschner-wire (10) when the proximal sections (22.2, 24.2) are electrically and mechanically disconnected from the respective distal sections (22.1, 24.1) of the electrically conducting signal lines (22, 24).

2. The Kirschner-wire (10) of claim 1, wherein the connector (30) comprises two electrically conducting ring contact elements (32, 34) that are arranged at or close to the proximal end of the Kirschner-wire (10), said ring contact elements (32, 34) being spaced apart from each other in a longitudinal direction of the Kirschner-wire (10) and being electrically insulated from the Kirschner-wire (10), each ring contact element (32, 34) being electrically connected to a respective one of the proximal sections (22.1., 24.1) of the electrically conducting signal lines (22, 24).

3. The Kirschner-wire (10) of claim 1 or 2, wherein the Kirschner-wire (10) is hollow and has a lumen (14) and wherein the localizer coil (20) is arranged within said lumen (14).

4. The Kirschner-wire (10) of one of claims 1 to 3, wherein the Kirschner-wire (10) has an outer diameter of between 1.5 mm and 2.5 mm.

5. The Kirschner-wire (10) of one of claims 1 to 4, wherein the localizer coil (20) has an outer diameter of less than 1 mm, preferably less than 0.5 mm, such as 0.45 mm.

6. The Kirschner-wire of claim 5, wherein the localizer coil comprises an outer diameter of between about 0.5 mm and about 0.45 mm.
